# EUROPÄISCHE PATENTSCHRIFT

(11) **EP 0 694 513 B1**
(45) Veröffentlichungstag und Bekanntmachung des Hinweises auf die Patenterteilung: **28.10.1998**
(21) Anmeldenummer: 95106074.8
(22) Anmeldetag: 24.04.1995
(51) Int. Cl.: C07C 17/363, C07C 25/06

(54) **Verfahren zur Herstellung von Chloraromaten**
Process for preparing chlorinated aromatic compounds
Procédé pour la préparation des composés aromatiques chlorés

(30) Priorität: 05.05.1994 DE 4415777
(43) Veröffentlichungstag der Anmeldung: 31.01.1996
(73) Patentinhaber: BAYER AG, 51368 Leverkusen (DE)
(72) Erfinder: Lui, Norbert, Dr., D-51061 Köln (DE); Marhold, Albrecht, Dr., D-51373 Leverkusen (DE)

(56) Entgegenhaltungen:
- DE-A- 4 225 763

## Beschreibung

Die vorliegende Erfindung betrifft ein Flüssigphasen-Verfahren zur Herstellung von Chloraromaten aus aromatischen Chlorameisensäureestern.

Es ist bekannt, daß sich aliphatische Chlorameisensäureester in flüssiger Phase in Gegenwart von Lewis-Säuren thermisch zu den entsprechenden Chloralkanen umsetzen lassen (siehe DE-OS 2 931 777, US-PS 4 814 524 und DE-PS 857 350).

Versuche diese Reaktion auf aromatische Chlorameisensäureester zu übertragen zeigten, daß aromatische Chlorameisensäureester in anderer Weise reagieren als aliphatische Halogenameisensäureester. Aromatische Chlorameisensäureester reagieren in Gegenwart von aromatischen Lösungsmitteln und Lewis-Säuren beim Erhitzen nicht unter Decarboxylierung zu Chloraromaten, sondern in einer Friedel-Crafts-Reaktion zu Phenylbenzoaten (J. Org. Chem. 22, 325 (1957)). Selbst wenn man in Abwesenheit aromatischer Lösungsmittel arbeitet, war deshalb damit zu rechnen, daß gegebenenfalls durch Decarboxylierung entstehendes Chlorbenzol sich sofort mit noch vorhandenem aromatischem Chlorameisensäureester zu Phenylbenzoaten umsetzt und Chlorbenzol nicht isoliert werden kann.

Andere Verfahren zur Herstellung von substituierten Chloraromaten sind ebenfalls nachteilig. So liefert die direkte Chlorierung von Alkylaromaten nur schwierig auftrennbare Isomerengemische, welche das gewünschte Isomere häufig nur zu einem geringen Anteil enthalten (J. org. Chem. 55, 5260 bis 5269 (1990)).

Aromatische Chlorameisensäureester lassen sich auch in Gegenwart von Aluminiumoxid, das gegebenenfalls mit Edelmetallen belegt ist, in der Gasphase in Halogenaromaten umwandeln (EP-OS 188 241 und 427 603).

Schließlich ist aus der DE-A 42 25 763 bekannt, daß man Chloraromaten aus den entsprechenden Chlorameisensäureestern durch Erhitzen in Gegenwart von Fluorwasserstoff oder Lewis-Säuren herstellen kann. Die Ausbeuten liegen dabei zwischen 20 und 74 %, was häufig unbefriedigend ist.

Es wurde nun ein Verfahren zur Herstellung von Chloraromaten der Formel (I) 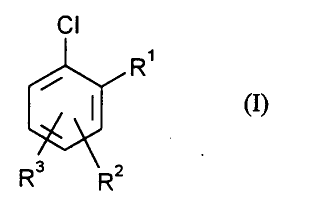 in der
- R¹: für C₁-C₆-Alkyl,
- R²: für Wasserstoff oder C₁-C₆-Alkyl und
- R³: für Wasserstoff, C₁-C₆-Alkyl, Fluor, Chlor oder Brom stehen,
gefunden,
das dadurch gekennzeichnet ist, daß man Chlorameisensäureester der Formel (II) 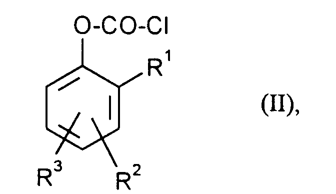 in der die verwendeten Symbole die bei Formel (I) angegebene Bedeutung haben,
in Gegenwart von Polychlorbenzolen als Lösungsmittel und einer katalytischen Menge einer oder mehrerer Lewis-Säuren aus der Gruppe, die Aluminiumhalogenide, Eisenhalogenide und Antimonhalogenide umfaßt, in flüssiger Phase auf 90 bis 240°C erhitzt.

Vorzugsweise stehen in den Formeln (I) und (II)
- R¹: für geradkettiges oder verzweigtes C₁-C₆-Alkyl oder cyclisches C₅-C₆-Alkyl,
- R²: für Wasserstoff, geradkettiges oder verzweigtes C₁-C₆-Alkyl oder cyclisches C₅-C₆-Alkyl und
- R³: für Wasserstoff, geradkettiges oder verzweigtes C₁-C₆-Alkyl, cyclisches C₅-C₆-Alkyl, Fluor oder Chlor.

Besonders bevorzugt stehen
- R¹ und R²: unabhängig voneinander für Methyl, Ethyl, n-Butyl oder i-Propyl und
- R³: für Wasserstoff oder Methyl.

Es ist weiterhin bevorzugt, daß der Substituent R² nicht für Wasserstoff steht und die Substituenten R¹ und R² in 2- und 6-Position in Bezug auf Chlor vorliegen. In diesen Fällen werden Chloraromaten der Formel (I) in besonders hohen Ausbeuten erhalten.

Das erfindungsgemäße Verfahren ist besonders für den Einsatz von 2,6-Dimethylphenylchlorameisensäureester geeignet.

Die zur Durchführung des erfindungsgemäßen Verfahrens als Ausgangsverbindungen benötigten Chlorameisensäureester der Formel (II) sind bekannt oder können analog zu bekannten Verbindungen hergestellt werden.

Als Lewis-Säuren aus der Gruppe umfassend Aluminiumhalogenide, Eisenhalogenide und Antimonhalogenide sind beispielsweise Aluminiumtrichlorid, Eisentrichlorid und mit Chlorwasserstoff vorbehandeltes Aluminiumoxid geeignet. Zur Umsetzung der Chlorameisensäureester zu Chloraromaten verwendet man vorteilhafterweise Aluminiumtrichlorid.

Die Lewis-Säuren können z.B. in Mengen von 0,01 bis 50 Mol-%, vorzugsweise 0,1 bis 25 Mol-%, bezogen auf den Chlorameisensäureester der Formel (II), eingesetzt werden.

Bevorzugte Polychlorbenzole sind Tri- und Tetrachlorbenzole wie 1,2,3-Trichlorbenzol, 1,2,4-Trichlorbenzol und 1,2,4,5-Tetrachlorbenzol.

Bei Batch-Fahrweise kann die Lösungsmittelmenge z.B. mindestens 25 Gew.-% der Eduktmenge betragen. Nach oben ist die Lösungsmittelmenge nicht begrenzt. Aus wirtschaftlichen Erwägungen wird man im allgemeinen nicht mehr als die 10-fache Gewichtsmenge Lösungsmittel (bezogen auf Edukt) einsetzen. Vorzugsweise setzt man bei Batch-Fahrweise 50 bis 500 Gew.-% Lösungsmittel (bezogen auf Edukt) ein.

Bei kontinuierlicher Fahrweise kann die Lösungsmittelmenge gegebenenfalls reduziert werden. Sie kann z.B. 2 bis 300 Gew.-%, bezogen auf die gesamte, während eines kontinuierlichen durchgeführten Arbeitscycluses eingeleitete Eduktmenge, betragen. Vorzugsweise liegt diese Menge bei 5 bis 30 Gew.-%.

Man kann das erfindungsgemäße Verfahren beispielsweise diskontinuierlich durchführen, indem man das Lösungsmittel und die Lewis-Säure in einem Reaktionsgefäß vorlegt und dann bei Reaktionstemperatur den Chlorameisensäureester zudosiert. Man kann auch kontinuierlich arbeiten, z.B. indem man den Chlorameisensäureester der Formel (II) bei solchen Druck- und Temperaturbedingungen in ein Gemisch aus Lösungsmittel und Lewis-Säure kontinuierlich eindosiert, daß der gebildete Chloraromat der Formel (I) kontinuierlich abdestilliert.

Hinsichtlich der Reaktionstemperatur ist zu beachten, daß diese innerhalb des Bereichs 90 bis 240°C mindestens so hoch gewählt wird wie die Temperatur, bei der das jeweilige Edukt beginnt zu decarboxylieren. Diese Mindesttemperatur kann gegebenenfalls durch routinemäßige Vorversuche leicht ermittelt werden. Höhere Temperaturen bis 240°C sind ohne Beschränkungen anwendbar.

Der Druck während der Durchführung des erfindungsgemäßen Verfahrens muß mindestens so hoch sein, daß sich das Edukt und das Lösungsmittel bei der jeweiligen Reaktionstemperatur in flüssiger Phase befinden. Nach oben hin ist der Druck nicht kritisch. Er kann z.B. bis zu 20 bar betragen.

Die Aufarbeitung bei diskontinuierlicher Arbeitsweise kann z.B. so vorgenommen werden, daß man nach Beendigung der Gasentwicklung den gebildeten Chloraromaten aus dem Reaktionsgemisch herausdestilliert. Das organische Lösungsmittel kann, z.B. durch Destillation, wieder gewonnen und erneut umgesetzt werden.

Das erfindungsgemäße Verfahren hat eine Reihe von Vorteilen. Es gestattet die Herstellung von Chloraromaten der Formel (I), insbesondere von 2,6-Dialkylchlor-benzolen, auf einfache Weise (einstufig) und in besonders guten Ausbeuten. Es kann in einfache Apparaturen und bei relativ niedrigen Temperaturen durchgeführt werden. Das Lösungsmittel kann recyclisiert werden. Während der Umsetzung bilden sich beim erfindungsgemäßen Verfahren weniger polymere und harzartige Nebenprodukte als bei anderen Verfahren.

Es ist überraschend, daß beim erfindungsgemäßen Verfahren trotz der Gegenwart von Lewis-Säuren Friedel-Crafts-Reaktionen nicht oder höchstens in ganz untergeordnetem Maß ablaufen.

### Beispiele

### Beispiel 1

Zu 235 g 1,2,3-Trichlorbenzol und 2 g AlCl₃ wurden bei 190°C 50 g 2,6-Dimethylphenylchlorameisensäureester in 20 min zugetropft. Nach Beendigung der Gasentwicklung wurde das 2,6-Dimethylchlorbenzol aus der Reaktionsmischung herausdestilliert. Ausbeute 94 %.

### Beispiel 2

Zu 7500 ml 1,2,4-Trichlorbenzol und 50 g AlCl₃ wurden bei 190°C 2280 g 2,6-Dimethylphenylchlorameisensäureester zugetropft und das 2,6-Dimethylchlorbenzol gleichzeitig aus der Reaktionsmischung herausdestilliert. Ausbeute 1690 g (97 % der Theorie) an 2,6-Dimethylchlorbenzol.

### Beispiel 3

Zu 433 g 1,2,4,5-Tetrachlorbenzol und 2 g AlCl₃ wurden bei 195°C 40 g 2,6-Dimethylphenylchlorameisensäureester in 20 min zugetropft. Nach Beendigung der Gasentwicklung wurde das 2,6-Dimethylchlorbenzol aus der Reaktionsmischung herausdestilliert. Ausbeute 95 %.

## Patentansprüche

1. Verfahren zur Herstellung von Chloraromaten der Formel (I) 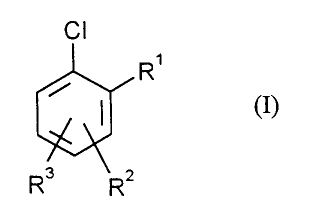 in der
R¹ für C₁-C₆-Alkyl,
R² für Wasserstoff oder C₁-C₆-Alkyl und
R³ für Wasserstoff, C₁-C₆-Alkyl, Fluor, Chlor oder Brom stehen,
dadurch gekennzeichnet, daß man Chlorameisensäureester der Formel (II) 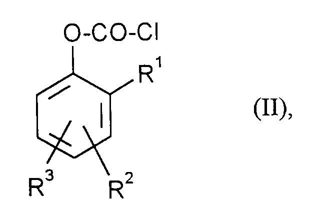 in der die verwendeten Symbole die bei Formel (I) angegebene Bedeutung haben,
in Gegenwart von Polychlorbenzolen als Lösungsmittel und einer katalytischen Menge einer oder mehrerer Lewis-Säuren aus der Gruppe, die Aluminiumhalogenide, Eisenhalogenide und Antimonhalogenide umfaßt, in flüssiger Phase auf 90 bis 240°C erhitzt.

2. Verfahren nach Anspruch 1, dadurch gekennzeichnet, daß in den Formeln (I) und (II)
R¹ für geradkettiges oder verzweigtes C₁-C₆-Alkyl oder cyclisches C₅-C₆-Alkyl,
R² für Wasserstoff, geradkettiges oder verzweigtes C₁-C₆-Alkyl oder cyclisches C₅-C₆-Alkyl und
R³ für Wasserstoff, geradkettiges oder verzweigtes C₁-C₆-Alkyl, cyclisches C₅-C₆-Alkyl, Fluor oder Chlor steht.

3. Verfahren nach Ansprüchen 1 und 2, dadurch gekennzeichnet, daß bei den Formeln (I) und (II) der Substituent R² nicht für Wasserstoff steht und die Substituenten R¹ und R² in 2- und 6-Position in Bezug auf Chlor vorliegen.

4. Verfahren nach Ansprüchen 1 bis 3, dadurch gekennzeic hnet, daß man pro Mol Chlorameisensäureester der Formel (II) 0,01 bis 50 Mol-% Lewis-Säuren aus der Gruppe, die Aluminiumhalogenide, Eisenhalogenide und Antimonhalogenide umfaßt, einsetzt.

5. Verfahren nach Ansprüchen 1 bis 4, dadurch gekennzeichnet, daß die Reaktionstemperatur mindestens so hoch ist wie die Temperatur, bei der das jeweilige Edukt beginnt zu decarboxylieren.

6. Verfahren nach Ansprüchen 1 bis 5, dadurch gekennzeichnet, daß der Druck mindestens so hoch ist, daß sich das Edukt und das Lösungsmittel bei der jeweiligen Reaktionstemperatur in flüssiger Phase befinden.

7. Verfahren nach Ansprüchen 1 bis 6, dadurch gekennzeichnet, daß man nach Beendigung der Reaktion aus dem Reaktionsgemisch den gebildeten Chloraromaten der Formel (I) durch Destillation abtrennt.

8. Verfahren nach Ansprüchen 1 bis 6, dadurch gekennzeichnet, daß man den jeweiligen Chlorameisensäureester der Formel (II) in ein Gemisch aus Lösungsmittel und Lewis-Säure kontinuierlich eindosiert und den gebildeten Chloraromaten der Formel (I) kontinuierlich abdestilliert.

9. Verfahren nach Ansprüchen 1 bis 8, dadurch gekennzeichnet, daß man 2,6-Dimethylchlorameisensäureester einsetzt.

## Claims

1. Process for the preparation of chlorinated aromatics of the formula (I) 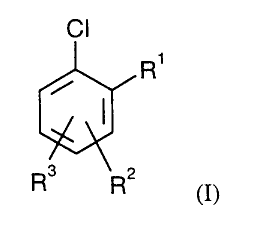 in which
R¹ represents C₁-C₆-alkyl,
R² represents hydrogen or C₁-C₆-alkyl and
R³ represents hydrogen, C₁-C₆-alkyl, fluorine, chlorine or bromine,
characterized in that esters of chloroformic acid of the formula (II) 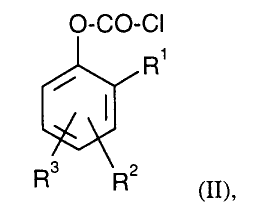 in which the symbols used have the meaning given under formula (I),
are heated in the liquid phase to 90 to 240°C in the presence of polychlorobenzenes as solvent and a catalytic amount of one or more Lewis acids selected from the group consisting of aluminium halides, iron halides and antimony halides.

2. Process according to Claim 1, characterized in that in the formulae (I) and (II)
R¹ represents straight-chain or branched C₁-C₆-alkyl or cyclic C₅-C₆-alkyl,
R² represents hydrogen, straight-chain or branched C₁-C₆-alkyl or cyclic C₅-C₆-alkyl and
R³ represents hydrogen, straight-chain or branched C₁-C₆-alkyl, cyclic C₅-C₆-alkyl, fluorine or chlorine.

3. Process according to Claims 1 and 2, characterized in that in the formulae (I) and (II) the substituent R² does not represent hydrogen and the substituents R¹ and R² are present in the 2- and 6-position in relation to chlorine.

4. Process according to Claims 1 to 3, characterized in that 0.01 to 50 mol % of Lewis acids selected from the group consisting of aluminium halides, iron halides and antimony halides is used per mole of chloroformic ester of the formula (II).

5. Process according to Claims 1 to 4, characterized in that the reaction temperature is at least as high as the temperature at which the particular starting material begins to decarboxylate.

6. Process according to Claims 1 to 5, characterized in that the pressure is at least high enough so that the starting material and the solvent are situated in the liquid phase at the particular reaction temperature.

7. Process according to Claims 1 to 6, characterized in that after completion of the reaction the chlorinated aromatic formed of the formula (I) is separated off from the reaction mixture by distillation.

8. Process according to Claims 1 to 6, characterized in that the particular chloroformic ester of the formula (II) is fed continuously into a mixture of solvent and Lewis acid and the chlorinated aromatic formed of the formula (I) is continuously distilled off.

9. Process according to Claims 1 to 8, characterized in that 2,6-dimethylphenyl chloroformate is used.

## Revendications

1. Procédé pour la préparation de composés aromatiques chlorés de la formule (I) 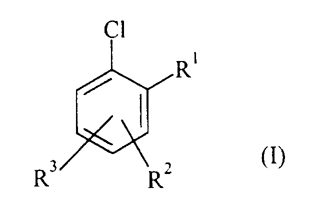 dans laquelle
R¹ représente un groupe alkyle en C₁-C₆,
R² représente un atome d'hydrogène ou un groupe alkyle en C₁-C₆, et
R³ représente un atome d'hydrogène, un groupe alkyle en C₁-C₆, un atome de fluor, de chlore ou de brome,
caractérisé en ce que l'on chauffe en phase liquide à de 90 à ,40°C un ester d'acide chloroformique de la formule (II) 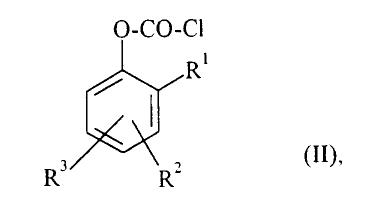 dans laquelle les symboles utilisés ont la signification indiquée dans la formule (I),
en présence de polychlorobenzènes comme solvant et d'une quantité catalytique d'un ou plusieurs acides de Lewis du groupe qui comprend les halogénures d'aluminium, les halogénures de fer et les halogénures d'antimoine.

2. Procédé selon la revendication 1, caractérisé en ce que dans les formules (I) et (II)
R¹ représente un groupe alkyle en C₁-C₆ linéaire ou ramifié ou un groupe alkyle en C₅-C₆ cyclique,
R² représente un atome d'hydrogène, un groupe alkyle en C₁-C₆ linéaire ou ramifié ou un groupe alkyle en C₅-C₆ cyclique et
R³ représente un atome d'hydrogène, un groupe alkyle en C₁-C₆ linéaire ou ramifié, un groupe alkyle en C₅-C₆ cyclique, un atome de fluor ou de chlore.

3. Procédé selon les revendications 1 et 2, caractérisé en ce que dans les formules (I) et (II) le substituant R² ne représente pas un atome d'hydrogène et les substituants R¹ et R² sont en position 2 et 6 par rapport au chlore.

4. Procédé selon les revendications 1 à 3, caractérisé en ce que l'on utilise par mol d'ester d'acide chloroformique de la formule (II) de 0,01 à 50% en mol d'acides de Lewis du groupe qui comprend les halogénures d'aluminium, les halogénures de fer et les halogénures d'antimoine.

5. Procédé selon les revendications 1 à 4, caractérisé en ce que la température de la réaction est au moins aussi élevée que la température à laquelle l'éduit correspondant commence à être décarboxylé.

6. Procédé selon les revendications 1 à 5, caractérisé en ce que la pression est au moins aussi élevée que l'éduit et le solvant sont en phase liquide à la température correspondante de la réaction.

7. Procédé selon les revendications 1 à 6, caractérisé en ce que l'on sépare à la fin de la réaction les composés aromatiques chlorés formés de la formule (I) du mélange réactionnel par distillation.

8. Procédé selon les revendications 1 à 6, caractérisé en ce que l'on ajoute en continu l'ester d'acide chloroformique correspondant de la formule (II) dans un mélange constitué de solvant et d'acide de Lewis et on élimine en continu par distillation les composés aromatiques chlorés formés de la formule (I).

9. Procédé selon les revendications 1 à 8, caractérisé en ce que l'on utilise un ester d'acide 2,6-diméthylchloroformique.
